(19) Europäisches Patentamt
European Patent Office
Office européen des brevets

(11) Veröffentlichungsnummer: **0 052 833**
**B1**

(12) # EUROPÄISCHE PATENTSCHRIFT

(45) Veröffentlichungstag der Patentschrift: **14.12.83**

(51) Int. Cl.³: **C 07 C 127/22,** A 01 N 47/34

(21) Anmeldenummer: **81109618.9**

(22) Anmeldetag: **11.11.81**

(54) **Harnstoffderivate, ihre Herstellung und Verwendung.**

(30) Priorität: **22.11.80 DE 3044055**
**15.06.81 DE 3123720**

(43) Veröffentlichungstag der Anmeldung:
**02.06.82 Patentblatt 82/22**

(45) Bekanntmachung des Hinweises auf die Patenterteilung:
**14.12.83 Patentblatt 83/50**

(84) Benannte Vertragsstaaten:
**AT BE CH DE FR GB IT LI LU NL**

(56) Entgegenhaltungen:
**DE - A - 3 003 112**

**Die Akte enthält technische Angaben, die nach dem Eingang der Anmeldung eingereicht wurden und die nicht in dieser Patentschrift enthalten sind.**

(73) Patentinhaber: **CELAMERCK GmbH & Co. KG, Binger Strasse 173, D-6507 Ingelheim am Rhein (DE)**

(72) Erfinder: **Becher, Heinz Manfred, Dr., Pfarrer-Heberer-Strasse 5, D-6530 Bingen (DE)**
Erfinder: **Prokic-Immel, Ricarda, Dr., Rubensallee 47, D-6500 Mainz (DE)**
Erfinder: **Wirtz, Walter, Dr., Reuterallee 12, D-6100 Darmstadt (DE)**

EP 0 052 833 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Harnstoffderivate, ihre Herstellung und Verwendung

Die Erfindung betrifft neue Harnstoffderivate der allgemeinen Formel

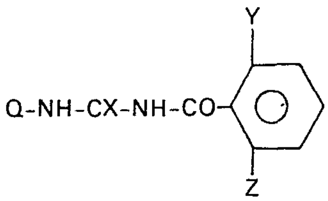

(I)

in der

Q für 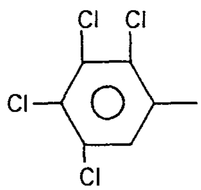 (II)

oder 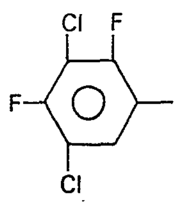 (III),

X für Sauerstoff oder Schwefel,
Y für Chlor oder Fluor und
Z für Wasserstoff, Chlor oder Fluor
steht, sowie die Herstellung dieser Verbindungen nach an sich bekannten Verfahren und die Verwendung bei der Bekämpfung von tierischen Schädlingen im Pflanzenschutz.

Aus der DE-AS 2 123 236 sind Benzoylharnstoffe mit Wirkung gegen tierische Schädlinge bekannt. Die in dieser DE-AS beschriebene Verbindung der Formel

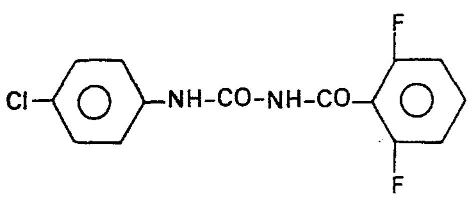

ist ein Handelsprodukt (Diflubenzuron). Gegenüber dieser Verbindung zeigen die Verbindungen gemäss der Erfindung eine deutlich überlegene Wirkung, z.B. gegen Insekten im Larvenstadium.

Die Herstellung von Verbindungen mit tetrahalogensubstituierter Phenylgruppe wird durch die Patentanmeldung auch nicht nahegelegt. Aus der Tabelle mit den Wirkungsangaben ergibt sich nämlich, dass für die Verbindung des Typs

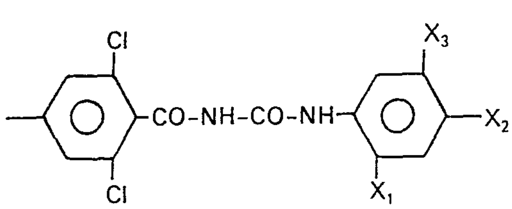

mit zunehmender Substitution die Wirkung schwächer wird (Spalte 7 Nr. 4 > Spalte 7 Nr. 1 > Spalte 9 Nr. 49). Auch wird in Spalte 6, Zeile 55 bis 59 der Auslegeschrift bemerkt, dass bei Verbindungen mit 1 oder 2 Substituenten in der Phenylgruppe die höchste Wirksamkeit gefunden wurde.

In der DE-A 3 003 112 werden Verbindungen beschrieben, die in der Phenylgruppe durch vier Fluoratome substituiert sind. Überraschenderweise sind jedoch die erfindungsgemässen Verbindungen, beispielsweise die Verbindungen nach Beispiel 2 und Beispiel 3, den bekannten Verbindungen in der Wirkungsstärke deutlich überlegen.

Hervorzuheben sind in diesem Zusammenhang Verbindungen, in denen X/Y/Z die Bedeutung O/F/F; O/Cl/F; O/Cl/H; S/F/F; S/Cl/F oder S/Cl/H haben, vor allem, wenn gleichzeitig Q 2,4-Difluor-3,5-dichlorphenyl bedeutet.

Die Herstellung der neuen Verbindungen kann nach an sich bekannten Verfahren erfolgen:

a) indem man das Anilin der Formel

$Q-NH_2$ (IV)

mit einem Isocyanat bzw. Senföl der Formel

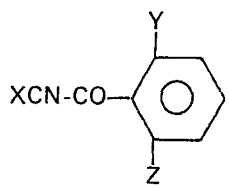

(V)

oder

b) ein Isocyanat bzw. Senföl der Formel

Q-NCX (VI)

mit einem Benzamid der Formel

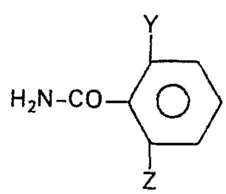

(VII)

umsetzt.

Die Umsetzungen gemäss a) und b) werden bei Temperaturen zwischen der Umgebungstemperatur und der Siedetemperatur des Reaktionsgemisches durchgeführt. Als Reaktionsmedium dient ein inertes Lösungsmittel, z.B. ein aromatischer Kohlenwasserstoff, etwa Toluol oder Xylol, Chlorbenzol, Pyridin, ein Äther wie Dioxan, Tetrahydrofuran, ggf. in Gegenwart einer tertiären organischen Base (Triäthylamin, Pyridin).

Die Ausgangsstoffe können, soweit sie noch nicht beschrieben wurden, nach üblichen Methoden in an sich bekannter Weise gewonnen werden.

Das Anilin der Formel IV erhält man aus der entsprechenden Nitroverbindung durch Reduktion der Nitrogruppe mit den hierfür gebräuchlichen Reduktionsmitteln. Aus dem Anilin IV entsteht durch Umsetzung mit Phosgen bzw. Thiophosgen das Isocyanat bzw. Senföl der Formel VI.

Benzoylisocyanate der Formel V können aus den entsprechenden Benzamiden und Oxalylchlorid erhalten werden, Benzoylisothiocyanate der Formel V aus den entsprechenden Benzoylchloriden und Kaliumrhodanid.

Die neuen Verbindungen können zur Bekämpfung von Insekten und Akariden eingesetzt werden. Sie eignen sich daher als Wirkstoffe für Insektizide und Akarizide, die gegen Schadinsekten und Milben sowie deren Entwicklungsstadien, wie Raupen und Larven eingesetzt werden können. Ausser schädlichen Akariden können besonders schädliche Insekten aus den Gruppen der Dipteren, Coleopteren, Lepidopteren, Hemipteren, Homopteren und Hymenopteren bekämpft werden. Als Beispiele seien im einzelnen genannt:

Stechmücken (Aedes), mexikanische Bohnenkäfer (Epilachna), Kartoffelkäfer (Leptinotasa), Kohlschabe (Plutella), Baumwollraupe (Prodenia), Spinnmilben (Tetranychus), Tarsonemus, Palpenmotte (Dichomeris), Kleinschmetterlinge (Pyroderces), Argyroplose ocellana, Frostspanner, Knospenwickler, Schalenwickler (Adoxophyes reticulana), Gespinstmotten, Eichenwickler, Traubenwickler, Springwurm, Maiszünsler, Baumwollkapselkäfer, Birnblattsauger, Rübenfliege. Für die Anwendung werden die erfindungsgemässen Wirkstoffe mit üblichen Hilfs- und/oder Trägerstoffen zu gebräuchlichen Formulierungen verarbeitet, z.B. Emulsionskonzentraten, Suspensionspulvern, Stäuben. Die Anwendung erfolgt in Spritzbrühen und Stäuben mit Wirkstoffkonzentrationen zwischen etwa 0,0005 und 2%, in Form von ULV-Formulierungen auch mit höheren Wirkstoffkonzentrationen (bis etwa 90%). Die Aufwandmenge pro Hektar beträgt je nach Wirkstoff und Kultur zwischen etwa 0,005 und 0,5 kg, vorzugsweise zwischen 0,01 und 0,25 kg.

Formulierungsbeispiel:
Suspensionspulver (Angaben in Gew.-%)

25% Wirkstoff gemäss der Erfindung
55% Kaolin
10% kolloidale Kieselsäure
9% Ligninsulfonat (Dispergiermittel)
1% Natriumtetrapropylenbenzolsulfonat (Netzmittel)

Die Bestandteile werden wie üblich zu einem Suspensionspulver (Partikelgrösse: >4 $\mu$) verarbeitet. Für die Anwendung wird mit Wasser eine Spritzbrühe hergestellt, die etwa 0,0005 bis 0,5 Gew.-% Wirkstoff enthält.

Die überlegene Wirksamkeit der erfindungsgemässen Verbindungen zeigt sich z.B. beim Vergleich mit dem oben erwähnten Handelsprodukt Diflubenzuron.

Der Vergleich wurde an Larven (Aedes aegyptii, A) und an Raupen (Spodoptera littoralis, B) im Labor durchgeführt. Ermittelt wurde die $LD_{95}$ in ppm Wirkstoff, wobei die Spritzbrühe aus einer 0,1–0,5-prozentigen Lösung der Wirkstoffe in Aceton durch Verdünnen mit Wasser hergestellt wurde.

I: N-(2,4-Difluor-3,5-dichlorphenyl)-N′-(2-chlor-6-fluorbenzoyl)-harnstoff
II: N-(2,4-Difluor-3,5-dichlorphenyl)-N′-(2-chlor-benzoyl)-thioharnstoff
III: N-(2,3,4,5-Tetrachlorphenyl)-N′-(2,6-difluorbenzoyl)-harnstoff
IV: N-(2,3,4,5-Tetrachlorphenyl)-N′-(2-chlorbenzoyl)-thioharnstoff
V: N-(2,3,4,5-Tetrachlorphenyl)-N′-(2-chlor-6-fluorbenzoyl)-harnstoff

| Wirkstoff | $LD_{95}$ gegen A [ppm Wirkstoff] | $LD_{95}$ gegen B [ppm Wirkstoff] |
|---|---|---|
| Diflubenzuron | 0,0031 | 3,4 |
| I | 0,0016 | 0,042 |
| II | – | 0,30 |
| III | 0,00084 | 0,3 |
| IV | – | 0,4 |
| V | 0,00094 | 0,65 |

Die Herstellung der neuen Verbindungen und verschiedener Ausgangsstoffe ist in den folgenden Beispielen näher erläutert:

A. Herstellung von Ausgangsstoffen

1. 2,4-Difluor-3,5-dichlor-nitro-benzol

In die Lösung von 261 g (1,0 Mol) 2,3,4,5-Tetrachlornitrobenzol in 1000 ml reinem, wasserfreiem Dimethylformamid[1] werden 250 g wasserfreies ca. 200°C heisses Kaliumfluorid[2] eingetragen. Das so entstandene Gemisch wird 15 Stunden lang bei 140°C gut gerührt. Anschliessend lässt man das Reaktionsgemisch auf 40°C abkühlen, saugt das Ungelöste ab und wäscht mit Dimethylformamid nach. Das Lösungsmittel der vereinigten Filtrate wird im Vakuum über eine Kolonne abdestilliert. Der Destillationsrückstand wird in 1,5 Ltr. Diisopropylether gelöst. Die so erhaltene Lösung wird 3mal mit je 250 ml gesättigter wässriger Natriumbicarbonat-Lösung ausgeschüttelt. Danach wird sie mit Magnesiumsulfat getrocknet und auf Rückstand eingeengt. Dieser wird im Wasserstrahlvakuum destilliert.

Ausbeute: 142 g (0,62 Mol; 62 % d.Th.)
Sdp.: 119 – 122°C/20 mbar

[1] getrocknet mit $P_2O_5$ und anschliessend mit Molekularsieb
[2] getrocknet durch mehrstündiges Glühen bei 600°C.

2. 2,4-Difluor-3,5-dichloranilin
In die Mischung von 114 g (0,50 Mol) 2,4-Difluor-3,5-dichlor-nitrobenzol, 2 Ltr. Wasser und 10 ml Essigsäure werden unter Rühren bei 95-100°C 140 g (2,5 Mol) Eisenpulver eingetragen. Das so erhaltene Reaktionsgemisch wird 8 Stunden lang bei 95-100°C gut verrührt.

Danach gibt man 50 ml 40%ige Natronlauge zu und destilliert das Gemisch mit Wasserdampf. Das entstandene Difluor-dichloranilin geht zusammen mit Wasser über. Nach beendeter Destillation wird es aus dem heterogenen Destillat durch Extraktion mit Äthylenchlorid isoliert. Das Lösungsmittel der organischen Phase wird abdestilliert und das zurückgebliebene Produkt im Wasserstrahlvakuum destilliert.

Ausbeute: 91,5 g (0,46 Mol; 92 % d.Th.)
Sdp.: 118-121°C/20 mbar

3. 2,4-Difluor-3,5-dichlorphenylisocyanat
In die Lösung von 39,6 g (0,20 Mol) 2,4-Difluor-3,5-dichloranilin in 400 ml Toluol wird unter gutem Rühren so lange Chlorwasserstoff eingeleitet, bis sich kein Niederschlag mehr abscheidet. Das so erhaltene heterogene Gemisch wird auf 5-10°C abgekühlt. Bei dieser Temperatur werden unter Rühren 40 g (0,4 Mol) Phosgen eingeleitet. Danach wird unter Rühren langsam aufgeheizt, so dass nach ca. 2 Stunden 100-105°C erreicht sind. Dann wird 7 Stunden lang bei dieser Temperatur gerührt. In dieser Zeit entsteht eine klare Lösung; Chlorwasserstoff wird frei. Anschliessend wird bei dieser Temperatur 1 Stunde lang Stickstoff eingeleitet, um das überschüssige Phosgen abzutreiben. Danach wird das Lösungsmittel, zuletzt im Vakuum, abdestilliert. Das zurückgebliebene Rohprodukt wird im Wasserstrahlvakuum destilliert.

Ausbeute: 35,4 g (0,158 Mol; 79 % d.Th.)
Sdp.: 100-104°C/20 mbar

4. 2,6-Difluorbenzoylisocyanat
Zu der Lösung von 31,4 g (0,20 Mol) 2,6-Difluorbenzamid in 250 ml Toluol werden 28 g (0,22 Mol) Oxalylchlorid zugetropft. Die so erhaltene Lösung wird 5 Stunden lang am Rückfluss gekocht. Dabei werden Chlorwasserstoff und Kohlenmonoxid frei. Danach wird das Lösungsmittel, zuletzt im Vakuum, abdestilliert. Das zurückgebliebene Produkt wird im Ölpumpenvakuum destilliert.

Ausbeute: 28,5 g (0,156 Mol; 77 % d.Th.)
Sdp.: 54-56°C / 0,7 mbar

Auf die prinzipiell gleiche Weise sind ebenfalls zugänglich:

2,6-Dichlorbenzoylisocyanat;
Sdp. 75-78°C/0,13 mbar
2-Chlor-6-fluorbenzoylisocyanat;
Sdp. 75-78°C/0,13 mbar
2-Chlorbenzoylisocyanat;
Sdp. 73-77°C / 0,13 mbar

5. 2-Chlorbenzoylsenföl
In die Lösung von 52,5 g (0,30 Mol) o-Chlorbenzoyl-chlorid in 100 ml wasserfreiem Toluol werden 35 g (0,36 Mol) feingepulvertes, wasserfreies Kaliumrhodanid[3] eingetragen. Das so erhaltene Gemisch wird 6 Stunden lang am Rückfluss gekocht. Danach lässt man abkühlen, saugt das Ungelöste ab und engt das Filtrat auf Rückstand ein. Dieser wird im Wasserstrahlvakuum destilliert.

Ausbeute: 52 g (0,26 Mol; 87 % d.Th.)
Sdp.: 149-152°C/21 mbar

[3] Mehrstündiges Trocknen bei 110°C; anschliessend Verreiben.

B. Herstellung der Endprodukte der Formel I
Beispiel 1
N-(2,4-Difluor-3,5-dichlorphenyl)-N'-(2-chlorbenzoyl)-harnstoff
Die Mischung von 9,0 g (0,040 Mol) 2,4-Difluor-3,5-dichlorphenylisocyant, 6,5 g (0,042 Mol) 2-Chlorbenzamid und 100 ml Xylol wird 5 Stunden lang am Rückfluss gekocht. Danach lässt man abkühlen und saugt das ausgefallene Produkt ab und trocknet es.

Ausbeute: 14,3 g (0,0377 Mol; 94 % d.Th.)
Fp.: 225-229°C

Beispiel 2
N-(2,4-Difluor-3,5-dichlorphenyl)-N'-(2,6-difluorbenzoyl)-harnstoff
Zu der Lösung von 2,95 g (0,015 Mol) 2,4-Difluor-3,5-dichloranilin in 50 ml Toluol werden 3 g (0,016 Mol) 2,6-Difluor-benzoylisocyanat gegeben. Die so entstandene Lösung wird 15 Stunden lang bei Raumtemperatur gerührt. Danach wird das in dieser Zeit ausgefallene Produkt abgesaugt und getrocknet.

Ausbeute: 4,2 g (0,011 Mol; 73 % d.Th.)
Fp.: 221-224

Auf die prinzipiell gleiche Weise sind ebenfalls zugänglich:

a) N-(2,4-Difluor-3,5-dichlorphenyl)-N'-(2,6-dichlorbenzoyl)-harnstoff;
Fp. 238-242°C
b) N-(2,4-Difluor-3,5-dichlorphenyl)-N'-(2-chlor-6-fluorbenzoyl)-harnstoff;
Fp. 214-218°C
c) N-(2,4-Difluor-3,5-dichlorphenyl)-N'-(2-chlorbenzoyl)-harnstoff;
Fp. 228-232°C
d) N-(2,4-Difluor-3,5-dichlorphenyl)-N'-(2-chlorbenzoyl)-thioharnstoff;
Fp. 150-152°C

Beispiel 3

N-(2,3,4-5-Tetrachlor-phenyl)-N'-(2,6-difluorbenzoyl)-harnstoff

Zu der Lösung von 4 g (0,017 Mol) vic. Tetrachloranilin in 60 ml Toluol werden 3,9 g (0,020 Mol) 2,6-Difluorbenzoylisocyanat gegeben. Die entstandene Lösung wird 1 Stunde lang bei Raumtemperatur gerührt. Danach wird das in dieser Zeit ausgefallene Produkt abgesaugt, mit Toluol gewaschen und getrocknet.

Ausbeute: 7 g (0,017 Mol; prakt. quantitativ)
Fp.: 255–257°C.

Ein kleiner Teil wurde aus viel Aceton umkristallisiert.
Fp.: 257–259°C.
Entsprechend werden folgende Verbindungen erhalten:

a) N-(2,3,4,5-Tetrachlorphenyl)-N'-(2-chlor-6-fluorbenzoyl)-harnstoff; Fp. >250°C;
b) N-(2,3,4,5-Tetrachlorphenyl)-N'-(2-chlorbenzoyl)-thioharnstoff; Fp. 186–187°C;
c) N-(2,3,4,5-Tetrachlorphenyl)-N'-(2-chlorbenzoyl)-harnstoff; Fp. 247–248°C;
d) N-(2,3,4,5-Tetrachlorphenyl)-N'-(2,6-difluorbenzoyl)-thioharnstoff;
e) N-(2,3,4,5-Tetrachlorphenyl)-N'-(2-chlor-6-fluorbenzoyl)-thioharnstoff;
f) N-(2,3,4,5-Tetrachlorphenyl)-N'-(2-fluorbenzoyl)-harnstoff;
g) N-(2,3,4,5-Tetrachlorphenyl)-N'-(2-fluorbenzoyl)-thioharnstoff;
h) N-(2,3,4,5-Tetrachlorphenyl)-N'-(2,6-dichlorbenzoyl)-harnstoff; Fp. >260°C;
i) N-(2,3,4,5-Tetrachlorphenyl)-N'-(2,6-dichlorbenzoyl)-thioharnstoff.

## Patentansprüche

1. Harnstoffderivate der Formel

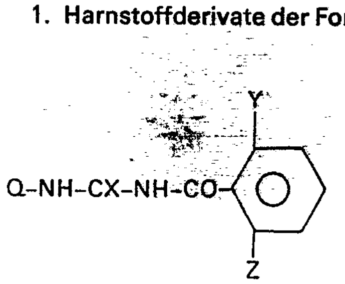

(I)

in der

Q für

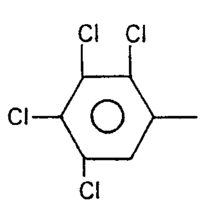

(II)

oder

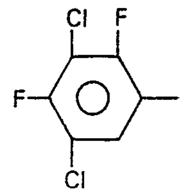

(III),

X für Sauerstoff oder Schwefel
Y für Chlor oder Fluor und
Z für Wasserstoff, Chlor oder Fluor
steht.

2. Schädlingsbekämpfungsmittel, gekennzeichnet durch einen Gehalt an mindestens einer Verbindung nach Anspruch 1.

3. Verwendung von Verbindungen nach Anspruch 1 bei der Bekämpfung von Insekten und Akariden, insbesondere von Schädlingen im Raupen- oder Larvenstadium.

4. Verfahren zur Herstellung von Verbindungen nach Anspruch 1, dadurch gekennzeichnet, dass man

a) das Anilin der Formel

$Q{-}NH_2$ (IV)

mit einem Isocyanat oder Senföl der Formel

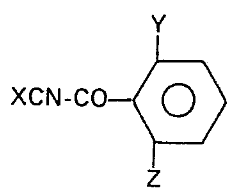

(V)

oder

b) ein Isocyanat oder Senföl der Formel

Q–NCX (VI)

mit einem Benzamid der Formel

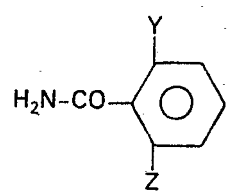

(VII)

umsetzt.

5. Verbindungen nach Anspruch 1, worin X/Y/Z die Bedeutungen O/F/F; O/Cl/F; O/Cl/H; S/F/F; S/Cl/F oder S/Cl/H besitzen.

6. Verbindungen nach Anspruch 1 oder 5, worin Q für 2,4-Difluor-3,5-dichlorphenyl steht.

7. Schädlingsbekämpfungsmittel nach Anspruch 2, insbesondere zur Bekämpfung von Insekten und Akariden, gekennzeichnet durch einen Gehalt an mindestens einer Verbindung nach Anspruch 5 oder 6.

8. Verwendung einer Verbindung nach Anspruch 5 oder 6 bei der Bekämpfung tierischer

Schädlinge, insbesondere von Insekten und Akariden.

**Revendications**

1. Dérivés d'urée de formule

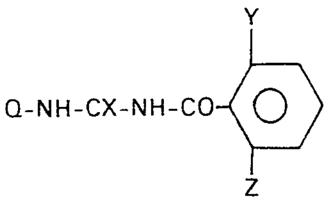
(I)

dans laquelle
Q remplace

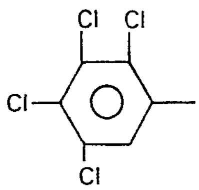
(II)

ou

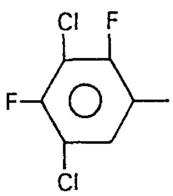
(III)

X l'oxygène ou le soufre,
Y le chlore ou le fluor, et
Z l'hydrogène, le chlore ou le fluor.

2. Agent de destruction des parasites, caractérisé par une teneur en au moins un composé selon la revendication 1.

3. Utilisation de composés selon la revendication 1 dans la destruction des insectes et acariens, en particulier des parasites au stade des chenilles ou des larves.

4. Procédé pour la préparation de composés selon la revendication 1, caractérisé en ce qu'on fait réagir

a) l'aniline de formule

$Q\text{-}NH_2$ (IV)

avec un isocyanate ou de l'huile de moutarde de formule

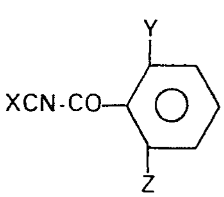
(V)

ou

b) un isocyanate ou de l'huile de moutarde de formule

Q-NCX (VI)

avec un benzamide de formule

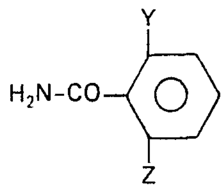
(VII)

5. Composés selon la revendication 1 dans lesquels X/Y/Z possèdent les significations O/F/F; O/Cl/F; O/Cl/H; S/F/F; S/Cl/F ou S/Cl/H.

6. Composés selon la revendication 1 ou 5, dans lesquels Q remplace 2,4-difluoro-3,5-dichlorophényle.

7. Agent de destruction des parasites selon la revendication 2, en particulier pour détruire les insectes et acariens, caractérisé par une teneur en au moins un composé selon la revendication 5 ou 6.

8. Utilisation d'un composé selon la revendication 5 ou 6 dans la destruction des parasites animaux, en particulier des insectes et acariens.

**Claims**

1. Urea derivatives of formula

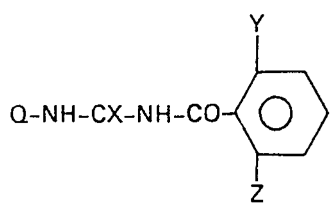
(I)

wherein
Q represents

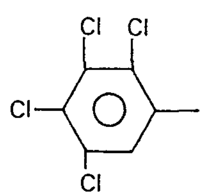
(II)

or

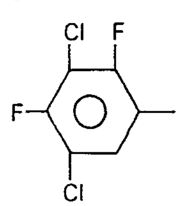
(III)

X represents oxygen or sulphur,
Y represents chlorine or fluorine and
Z represents hydrogen, chlorine or fluorine.

2. Pesticides, characterised in that they contain at least one compound as claimed in claim 1.

3. Use of compounds as claimed in claim 1 for controlling insects and acarids, particularly pests in the caterpillar or larval stage.

4. Process for preparing compounds as claimed in claim 1, characterised in that

a) the aniline of formula

$Q\text{-}NH_2$ (IV)

is reacted with an isocyanate or isothiocyanate of formula

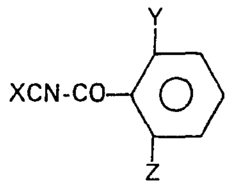

(V)

or

b) an isocyanate or isothiocyanate of formula

Q-NCX (VI)

is reacted with a benzamide of formula

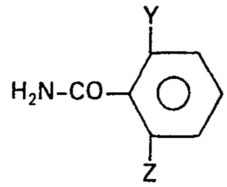

(VII)

5. Compounds as claimed in claim 1, wherein X/Y/Z have the meanings O/F/F; O/Cl/F; O/Cl/H; S/F/F; S/Cl/F or S/Cl/H.

6. Compounds as claimed in claim 1 or 5, wherein Q represents 2,4-difluoro-3,5-dichlorophenyl.

7. Pesticides as claimed in claim 2, more particularly for controlling insects and acarids, characterised in that they contain at least one compound as claimed in claim 5 or 6.

8. Use of a compound as claimed in claim 5 or 6 for controlling animal pests, particularly insects and acarids.